# EUROPEAN PATENT APPLICATION

(11) **EP 4 162 861 A1**
(43) Date of publication of application: **12.04.2023**
(21) Application number: 21818429.9
(22) Date of filing: 21.05.2021
(51) Int. Cl.: A61B 3/16

(54) **NON-CONTACT TONOMETER AND TONOMETER CONTROL PROGRAM**

(30) Priority: 03.06.2020 JP 2020097206; 30.06.2020 JP 2020113563
(71) Applicant: Nidek Co., Ltd., Gamagori-shi, Aichi, 443-0038 (JP)
(72) Inventor: HAMAGUCHI, Koji, Gamagori-shi Aichi 443-0038 (JP); SHIMIZU, Kazunari, Gamagori-shi Aichi 443-0038 (JP); SUGIMOTO, Yoshihiko, Gamagori-shi Aichi 443-0038 (JP); KOHCHIYAMA, Kazunori, Gamagori-shi Aichi 443-0038 (JP); UEMURA, Tsutomu, Gamagori-shi Aichi 443-0038 (JP)
(74) Representative: Zimmermann, Tankred Klaus
(86) International application number: PCT/JP2021/019455
(87) International publication number: WO 2021/246206

(57) **Abstract**

A technical issue is to provide a non-contact tonometer that can suck in fluid on a timely basis, and a tonometer control program. A non-contact tonometer measures eye pressure in an examinee's eye in a non-contact manner, and includes: a fluid ejection means for ejecting fluid to a cornea of the examinee's eye; a driving means for driving the fluid ejection means; and a control means for controlling the driving means, and the control means changes a timing of starting a fluid sucking operation after ejection of the fluid. As a result, it is possible to suck in the fluid on a timely basis.

## Description

### TECHNICAL FIELD

The disclosure relates to a non-contact tonometer that measures eye pressure in an examinee's eye in a non-contact manner, and a tonometer control program.

### BACKGROUND ART

A non-contact tonometer is known in which fluid such as air is ejected from a nozzle toward an examinee's eye, and then a predetermined deformed shape (for example, an applanated state) of the cornea caused by the ejected fluid is detected to measure eye pressure. In such an apparatus, a driving current is supplied to a driving device such as a solenoid, and a piston inside a cylinder is pressed forward with a driving force of the driving device, thereby blowing the fluid to the cornea of the examinee's eye to deform the cornea. Moreover, it is configured in such a manner that after the fluid is ejected to the examinee's eye, the piston is returned to an initial position thereof and the fluid is sucked into the cylinder to be ready for the next eye pressure measurement.

### CITATION LIST

### PATENT LITERATURE

PATENT LITERATURE 1: JP-A-03-118034
PATENT LITERATURE 2: JP-A-2004-89455

### SUMMARY OF INVENTION

However, a first problem of the known apparatus is that there is room for improvement in the timing of sucking the fluid into the cylinder after the ejection of the fluid. Moreover, a second problem of the known apparatus is that a foreign substance such as an aerosol that has been flown by the ejection of the fluid results in being sucked into the apparatus in some cases.

The present disclosure has a technical issue in providing a non-contact tonometer and tonometer control program that can suck in fluid on a timely basis, considering the first problem, or in providing a non-contact tonometer and tonometer control program that can restrain a foreign substance such as an aerosol flown by ejection of the fluid from being sucked in, considering the second problem.

In order to solve the above issues, the present disclosure is characterized by the following configurations:
(1) A non-contact tonometer measures eye pressure in an examinee's eye in a non-contact manner, and includes: a fluid ejection means for ejecting fluid to a cornea of the examinee's eye; a driving means for driving the fluid ejection means; and a control means for controlling the driving means. The control means changes a timing of starting a fluid sucking operation after ejection of the fluid.
(2) A non-contact tonometer measures eye pressure in an examinee's eye in a non-contact manner, and includes: a fluid ejection means for ejecting fluid to a cornea of the examinee's eye; a driving means for driving the fluid ejection means; and a control means for controlling the driving means. The control means waits until a possibility of sucking in a foreign substance after ejection of the fluid is reduced, and then starts a fluid sucking operation.
(3) A tonometer control program is to be executed in a non-contact tonometer that measures eye pressure in an examinee's eye in a non-contact manner, the tonometer control program being executed by a control means of the non-contact tonometer, thereby causing the non-contact tonometer to execute: a driving step of driving a fluid ejection means for ejecting fluid to a cornea of the examinee's eye; and a changing step of changing a timing of starting a fluid sucking operation after ejection of the fluid.
(4) A non-contact tonometer measures eye pressure in an examinee's eye in a non-contact manner, and includes: a fluid ejection means for ejecting fluid to a cornea of the examinee's eye; a driving means for driving the fluid ejection means; and a control means for controlling the driving means. The control means performs a first ejection for deforming the cornea during eye pressure measurement and, after the first ejection, performs a second ejection for removing a foreign substance near an outlet of the fluid ejection means before a next measurement.
(5) A tonometer control program is to be executed in a non-contact tonometer that measures eye pressure in an examinee's eye in a non-contact manner, the tonometer control program being executed by a control means of the non-contact tonometer, thereby causing the non-contact tonometer to execute: a driving step of driving a fluid ejection means for ejecting fluid to a cornea of the examinee's eye; a first ejection step of performing a first ejection for deforming the cornea during eye pressure measurement; and a second ejection step of, after the first ejection, performing a second ejection for removing a foreign substance near an outlet of the fluid ejection means before a next measurement.

### BRIEF DESCRIPTION OF DRAWINGS

Fig. 1 is a diagram illustrating an external configuration of the example.
Fig. 2 is a diagram illustrating an internal configuration of the example.
Fig. 3 is a diagram illustrating an optical system of the example.
Fig. 4 is a flowchart illustrating a control operation of the example.
Fig. 5 is a flowchart illustrating a control operation of a fluid ejection device.
Fig. 6 is a diagram illustrating a setting screen of a standby time displayed on a display device.
Fig. 7 is a diagram illustrating a mode setting screen displayed on the display device.
Fig. 8 is a flowchart illustrating a control operation of a fluid ejection device of a second example.
Fig. 9 is a diagram illustrating a measurement screen displayed on a display device.
Fig. 10 is a diagram illustrating a setting screen displayed on the display device.

### DESCRIPTION OF EMBODIMENTS

### <First Embodiment>

A first embodiment according to the present disclosure is described below. A non-contact tonometer of the first embodiment measures eye pressure in an examinee's eye in a non-contact manner. The non-contact tonometer includes, for example, a fluid ejection device (for example, a fluid ejection device 200), a driving device (for example, a solenoid 203), and a controller (for example, a controller 80). The fluid ejection device, for example, ejects fluid to the cornea of the examinee's eye. The driving device, for example, drives the fluid ejection device. The controller, for example, controls the driving device. The controller, for example, changes the timing of starting a fluid sucking operation after the ejection of the fluid. For example, the controller switches between a first timing preset to start the fluid sucking operation and a second timing that it takes longer to reach than the first timing. The non-contact tonometer of the first embodiment includes the above configuration and therefore can suck the fluid into the fluid ejection device on a timely basis. Consequently, it is possible to restrain, for example, the lacrimal fluid or dust of the examinee, or bacteria in the air from being sucked into the fluid ejection device.

The controller may change the setting of a standby time after the ejection of the fluid to change the timing of starting the fluid sucking operation. For example, the controller may change the above-mentioned second timing freely. For example, the controller may change the setting of the standby time after the ejection of the fluid in units of seconds. The controller may start the fluid sucking operation after the standby time of which setting has been changed has elapsed. In this manner, waiting for at least several seconds after the ejection of the fluid allows performing the sucking operation in a reliably ventilated state, and reducing the possibility of sucking in a foreign substance. The present inventors have experimentally verified that it is preferable to wait for two or more seconds. Moreover, the standby time is set to change the timing of starting the fluid sucking operation; therefore, the fluid can be sucked in at steady timings regardless of each measurement situation.

The non-contact tonometer may further include an operation accepter (for example, the controller 80). The operation accepter accepts, for example, an examiner's operation. In this case, the controller may change the timing of starting the fluid sucking operation on the basis of the examiner's operation accepted by the operation accepter during standby after the ejection of the fluid. Consequently, the examiner can cause the fluid sucking operation to be performed at a timing appropriate to each measurement.

The non-contact tonometer may further include a detector (for example, a chin rest sensor 3c, a face photographing device 90, a CCD camera 35, a position sensor, and a range sensor). The detector, for example, indirectly detects the possibility of sucking in a foreign substance. In this case, the controller may start the fluid sucking operation on the basis of the result of the detection by the detector. The detector may detect, for example, the presence or absence of the examinee, the blink of the examinee, or the position of a measuring unit.

The controller may switch between a first mode (for example, a manual change mode) that changes the timing of starting the fluid sucking operation and a second mode (for example, a time setting mode) that changes the timing of starting the fluid sucking operation on the basis of a condition different from the first mode. Consequently, the examiner can make a measurement in a favorite change mode.

The controller may switch whether or not to change the timing of starting the fluid sucking operation. For example, the controller may be configured in such a manner as to be capable of turning OFF a function of changing the timing of the fluid sucking operation on the basis of the examiner's selection based on the operation of an operating unit. Consequently, an appropriate measurement can be made in each measurement scene.

The controller may start the fluid sucking operation after waiting until the possibility of sucking in a foreign substance after the ejection of the fluid is reduced. Consequently, it is possible to restrain, for example, the lacrimal fluid or dust of the examinee, or bacteria in the air from being sucked into the fluid ejection device.

A processor of the non-contact tonometer may be caused to execute a tonometer control program stored in, for example, a storage. The tonometer control program includes, for example, a driving step and a change step. The driving step is the step of driving the fluid ejection device. The change step is the step of changing the timing of starting the fluid sucking operation after the ejection of the fluid.

### <Second Embodiment>

A second embodiment according to the present disclosure is described below. A non-contact tonometer of the second embodiment measures eye pressure in an examinee's eye in a non-contact manner. The non-contact tonometer of the second embodiment includes, for example, a fluid ejection device (for example, a fluid ejection device 200), a driving device (for example, a solenoid 203), and a controller (for example, a controller 80). The fluid ejection device ejects fluid to the cornea of the examinee's eye. The driving device drives the fluid ejection device. The controller controls the driving device. For example, in eye pressure measurement, the controller performs a first ejection (first ejection control) for deforming the cornea and, after the first ejection, performs a second ejection (second ejection control) for removing a foreign substance near an outlet of the fluid ejection device before the next measurement. In this manner, the non-contact tonometer of the second embodiment performs the second ejection after the first ejection; therefore, it is possible to restrain a foreign substance such as an aerosol flown by the first ejection from being sucked into the fluid ejection device.

The controller may perform the second ejection before completing a fluid sucking operation. For example, the controller may perform the second ejection even after the fluid sucking operation has been started, as long as it is before the sucking operation is completed.

The fluid ejection device may include a cylinder (for example, a cylinder 201), and a piston (for example, a piston 202) that moves in the cylinder. In this case, the controller may bias the piston, which is moving in a forward direction of movement in the first ejection, in a backward direction of movement, thereby slowing down or stopping the piston, and again bias the piston further in the forward direction of movement from the position (the position after the piston has slowed down or stopped) (increase the speed of the piston in the forward direction of movement), thereby performing the second ejection.

The pressure of the fluid that is ejected in the second ejection may be lower than the pressure of the fluid that is ejected in the first ejection. Consequently, it is possible to blow off, for example, an aerosol near the outlet without generating, for example, a new aerosol.

The controller may cause a display device (for example, a display device 85) to display information that the second ejection is being performed. Consequently, an examiner can confirm that the second ejection is being performed. Moreover, the examiner can explain to, for example, the examinee that the second ejection is being performed.

The controller may change, for example, the amount, pressure, or timing of ejection of the fluid in the second ejection. Moreover, the controller may be able to switch between the performance and non-performance of the second ejection. Consequently, the second ejection can be performed with an appropriate setting according to each examiner or each facility.

The controller may change the timing of starting the fluid sucking operation after the ejection of the fluid. Consequently, it is possible to stagger the timing when a foreign substance such as an aerosol flies after the ejection of the fluid and the timing of the sucking of the fluid and to reduce the possibility of sucking in a foreign substance such as an aerosol.

The controller may execute a tonometer control program stored in, for example, a storage. The tonometer control program may include, for example, a driving step, a first ejection step, and a second ejection step. The driving step is, for example, the step of driving the fluid ejection device that ejects the fluid to the cornea of the examinee's eye. The first ejection step is, for example, the step of performing the first ejection for deforming the cornea in eye pressure measurement. The second ejection step is the step of, after the first ejection, performing the second ejection for removing a foreign substance near the outlet of the fluid ejection device before the next measurement.

### <First Example>

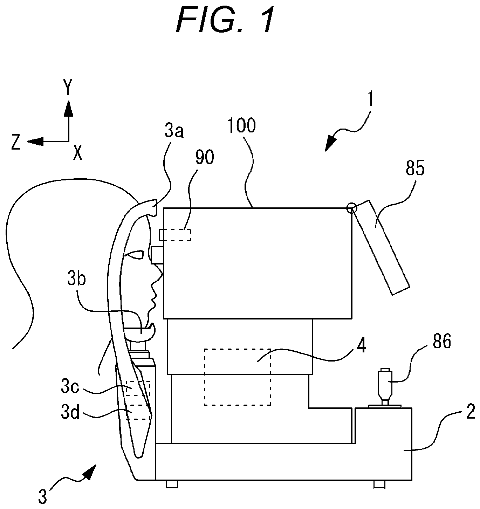

A non-contact tonometer of a first example is described below on the basis of the drawings. The non-contact tonometer of the first example measures eye pressure in an examinee's eye in a non-contact manner. The non-contact tonometer, for example, ejects fluid to the cornea of the examinee's eye, and measures eye pressure in the examinee's eye from a relationship between the deformed state of the cornea and the pressure of the fluid at this point in time. The non-contact tonometer includes, for example, a fluid ejection device, a driving device, and a controller. In Figs. 1 to 3, an X direction represents the right-left direction, a Y direction represents the up-down direction, and a Z direction represents the front-back direction.

As illustrated in Fig. 1, a non-contact tonometer 1 may include a base 2, a face support 3, a driving device 4, a display device 85, and a face photographing device 90. The base 2 supports a measuring unit 100 in a movable manner. The face support 3 supports the face of the examinee. The face support 3 includes a forehead rest 3a, a chin rest 3b, a chin rest sensor 3c, and a chin rest driver 3d. The chin rest sensor 3c detects whether or not the chin is on the chin rest 3b. The chin rest driver 3d moves the chin rest 3b up and down to adjust the height. The driving device 4 moves the measuring unit 100 in the X-Y-Z direction (a three-dimensional direction) relative to the base 2. The display device 85 displays, for example, an observation image and a measurement result of the examinee's eye. The display device 85 may be provided, for example, integrally with the non-contact tonometer 1, or separately from the apparatus. The display device 85 may be able to be placed in such a manner that a display screen faces not only the examinee but also the examinee. Various operation instructions of the examiner or the examinee are inputted into the display device 85. The display device 85 may be used as an operating unit 86. In this case, the display device 85 is used for, for example, various settings of the non-contact tonometer 1, the start of measurement, and an operation for sucking in air. Various human interfaces such as a joystick, a mouse, a keyboard, a trackball, and a button may be used as the operating unit 86. The face photographing device 90 photographs, for example, the face of the examinee's eye. The face photographing device 90 photographs, for example, the face including at least one of the examinee's left and right eyes.

### <Fluid Ejection Device>

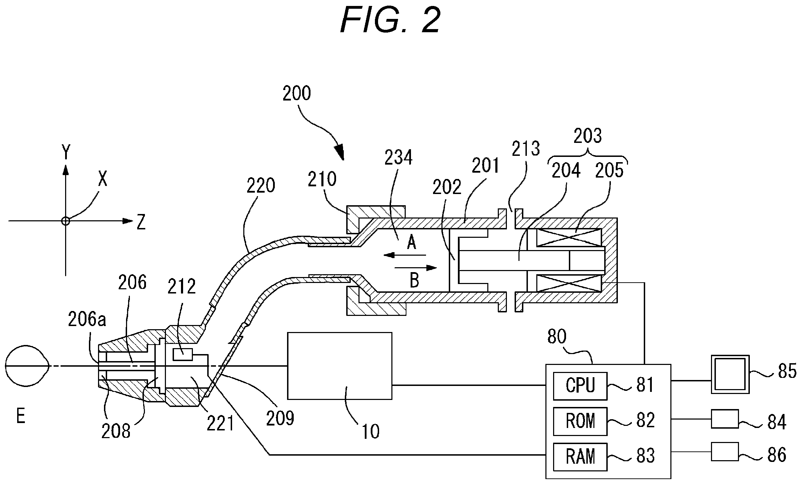

A fluid ejection device 200, for example, ejects fluid to the cornea of an examinee's eye E. The fluid ejection device 200 includes, for example, a cylinder 201, a piston 202, a solenoid actuator (hereinafter also referred to as the solenoid) 203, and a nozzle 206. The cylinder 201 and the piston 202 are used as an air compression mechanism that compresses air which is to be ejected to the examinee's eye. The cylinder 201 has, for example, a cylindrical shape. The piston 202 slides along an axial direction of the cylinder 201. The piston 202 compresses air in an air compression chamber 234 in the cylinder 201. The solenoid 203 of the example is what is called a direct acting solenoid, and operates linearly. The solenoid 203 includes a movable body 204 and a coil 205. A magnetic material such as a permanent magnet is used for the movable body 204. When an electric current flows through the coil 205, a magnetic field is generated inside the coil 205. The movable body 204 is moved in a direction A in Fig. 2 by an electromagnetic force received from the magnetic field. The movable body 204 is fastened to the piston 202 with, for example, an unillustrated screw, bolt, and nut. Therefore, the piston 202 moves together with the movable body 204. The movable body 204 moves, thereby moving the piston 202 in a compression direction (or a forward direction of movement or the direction A in Fig. 1). The nozzle 206 ejects the compressed air to the outside of the apparatus. The nozzle 206 includes an outlet 206a.

The piston 202 moves, thereby ejecting the fluid compressed in the air compression chamber 234 in the cylinder 201, from the nozzle 206 to the cornea of the examinee's eye E through a tube (or may be a pipe) 220 coupled to the tip of the cylinder 201 and through an airtight chamber 221 that stores the compressed air. For example, the cylinder 201 may be placed parallel to a horizontal plane (an XZ-plane), and the piston 202 may be moved horizontally by the driving of the solenoid 203 in the cylinder 201 to compress the fluid. For example, a longitudinal direction of the cylinder 201 is placed parallel to a horizontal direction, and the inner surface of the cylinder 201 guides the piston 202. Hence, the moving direction (compression direction) of the piston 202 is the horizontal direction. Each of the above configuration members is placed on a stage provided in a housing of an apparatus main body.

Moreover, the solenoid 203 of the example can change the moving direction of the movable body 204 by changing the direction of an electric current which is to be passed through the coil 205. For example, the movable body 204 moves in the compression direction (the forward direction of movement or the direction A in Fig. 2) when an electric current is passed in a forward direction through the coil 205, and moves in the opposite direction (a backward direction of movement or a direction B in Fig. 2) when an electric current is passed in a reverse direction. Therefore, the moving direction of the piston 202, which moves together with the movable body 204, can be changed by switching the direction of the electric current which is to be passed through the coil 205. For example, a forward current is passed through the coil 205, the piston 202 is moved in the direction A to compress the fluid in the air compression chamber 234, and then a reverse current is passed through the coil 205 to move the piston 202 in the direction B. As a result, the piston 202 can be returned to an initial position thereof.

The fluid ejection device 200 may include, for example, a glass plate 208 and a glass plate 209. The glass plate 208 is transparent, and holds the nozzle 206 and transmits observation light and alignment light. The glass plate 209 configures a back wall of the airtight chamber 221, and transmits the observation light and the alignment light.

The fluid ejection device 200 may include, for example, a pressure sensor 212, and an air bleeder hole 213. The pressure sensor 212, for example, detects pressure in the airtight chamber 221. The air bleeder hole 213, for example, allows reducing resistance before the piston 202 gains an initial velocity, and obtaining a rise in the change of pressure that is proportional with time.

### <Measuring Optical System>

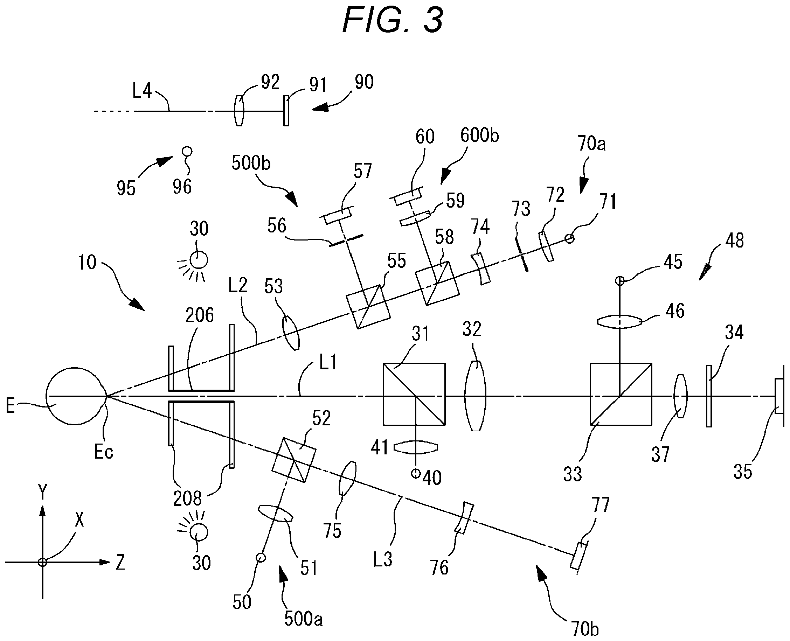

Fig. 3 is a schematic diagram of a measuring optical system 10 of the non-contact tonometer 1. An image of the examinee's eye illuminated by an infrared illumination light source 30 is formed on a CCD camera 35 through a beam splitter 31, an objective lens 32, a dichroic mirror 33, an imaging lens 37, and a filter 34. In other words, an optical system from the beam splitter 31 to the CCD camera 35 has an imaging device, and is used as an observing optical system for observing the anterior segment of the examinee's eye. In this case, an optical axis L1 is used as an observation optical axis.

The filter 34 transmits light from the light source 30 and an infrared light source 40 for alignment, and has the property of being impermeable to light from a light source 50 for detecting the deformation of the cornea and to visible light, which are described below. The image formed on the CCD camera 35 is displayed on the display device 85.

The infrared light projected from the light source 40 through a projection lens 41 is reflected by the beam splitter 31, and projected to the examinee's eye from the front. An image of a raster on the cornea formed on the corneal apex by the light source 40 is formed on the CCD camera 35 through the beam splitter 31 to the filter 34 to be used for alignment detection in the up-down and right-left directions. In other words, an optical system from the beam splitter 31 to the CCD camera 35 has an imaging device, and is used as a detecting optical system for detecting an alignment state in the up-down and right-left directions relative to the examinee's eye. In this case, the optical axis L1 is used as an alignment optical axis. In the example, the detecting optical system shares the observing optical system for observing the anterior segment.

A fixation optical system 48 includes the optical axis L1, and presents a fixation target to an eye E from the front. In this case, the optical axis L1 is used as a fixation optical axis. The fixation optical system 48 includes, for example, a visible light source (fixation lamp) 45, a projection lens 46, and the dichroic mirror 33, and projects, to the eye E, light for causing the eye E to fixate in the front direction. A light source such as an LED or laser is used for the visible light source 45. Moreover, for example, a two-dimensional display such as a liquid crystal display, in addition to a pattern light source such as a point light source, a slit light source, or a ring light source, is used for the visible light source 45.

Visible light emitted from the light source 45 passes through the projection lens 46, is reflected by the dichroic mirror 33, passes through the objective lens 32, and then is projected to the fundus of the eye E. Consequently, the eye E enters a state of fixating a fixation point in the front direction, and the direction of the visual line is fixed. The visible light emitted from the light source 45 passes through the projection lens 46 and the objective lens 32, thereby being converted to a parallel light flux.

A cornea deformation detecting optical system includes a light projecting optical system 500a and a light receiving optical system 500b, and is used to detect the deformed state of a cornea Ec. Each of the optical systems 500a and 500b is placed in the measuring unit 100, and moved three-dimensionally by the driving device 4.

The light projecting optical system 500a includes an optical axis L3 as a light projecting optical axis, and applies illuminating light diagonally to the cornea Ec of the eye E. The light projecting optical system 500a includes, for example, the infrared light source 50, a collimator lens 51, and a beam splitter 52. The light receiving optical system 500b includes a photosensor 57, and receives reflected light of the illuminating light from the cornea Ec of the eye E. The light receiving optical system 500b is placed substantially symmetrically about the optical axis L1 with respect to the light projecting optical system 500a. The light receiving optical system 500b includes, for example, a lens 53, a beam splitter 55, a pinhole plate 56, and the photosensor 57, and forms an optical axis L2 as a light receiving optical axis.

The light emitted from the light source 50 is converted to a substantially parallel light flux by the collimator lens 51, is reflected by the beam splitter 52, and then becomes coaxial (agrees) with the optical axis L3 of a light receiving optical system 70b described below to be projected to the cornea Ec of the examinee's eye. The light reflected from the cornea Ec becomes coaxial (agrees) with the optical axis L2 of a light projecting optical system 70a described below, passes through the lens 53, and then is reflected by the beam splitter 55, passes through the pinhole plate 56, and is received by the photosensor 57. The lens 53 is covered with a coating with the property of being impermeable to the light from the light sources 30 and 40. Moreover, the optical system for detecting the deformation of the cornea is placed in such a manner that the amount of light received by the photosensor 57 is at its maximum when the examinee's eye is in a predetermined deformed state (flat state).

Moreover, the cornea deformation detecting optical system also serves as a part of a first working distance detecting optical system, and a light projecting optical system of the first working distance detecting optical system shares the light projecting optical system 500a of the cornea deformation detecting optical system. A light receiving optical system 600b that receives light from the light source 50 reflected from the cornea Ec includes, for example, the lens 53 of the light projecting optical system 500a, a beam splitter 58, a condenser lens 59, and a position detector 60, and forms the optical axis L2 as a light receiving optical axis.

The illuminating light projected by the light source 50 and reflected from the cornea Ec forms a target image being a virtual image made by the light source 50. The light of the target image passes through the lens 53 and the beam splitter 55, is reflected by the beam splitter 58, passes through the condenser lens 59, and enters the one- or two-dimensional position detector 60 such as a PSD or line sensor. When the examinee's eye E (the cornea Ec) moves in a working distance direction (the Z direction), the target image made by the light source 50 also moves over the position detector 60 so that a control circuit 20 obtains working distance information on the basis of an output signal from the position detector 60. The output signal from the position detector 60 of the example is used for alignment (rough adjustment) in the working distance direction (the Z direction). The magnifying power of the light receiving optical system 600b of the first working distance detecting optical system is not as high as the magnifying power of the light receiving optical system 70b described below. Hence, the distance detection range of the position detector 60 in the Z direction is greater than that of a photodetector 77.

A corneal thickness measuring optical system includes the light projecting optical system 70a, the light receiving optical system 70b, and the fixation optical system 48, and is used to measure the corneal thickness of the examinee's eye E. Moreover, the light projecting optical system 70a shares a part of the cornea deformation detecting optical system and the first working distance detecting optical system.

The light projecting optical system 70a includes the optical axis L2 as a light projecting optical axis, and applies the illuminating light (measurement light) diagonally to the cornea Ec of the eye E. The light projecting optical system 70a includes, for example, an illuminating light source 71, a condenser lens 72, a light restriction member 73, a concave lens 74, and the lens 53 that is shared with the cornea deformation detecting optical system. A visible light source or infrared light source (including the near-infrared region) is used for the illuminating light source 71. For example, a light source such as an LED or laser is used. The condenser lens 72 condenses light emitted from the light source 71. The light sources 50 and 71 mutually use wavelength bands.

The light restriction member 73 is placed in an optical path of the light projecting optical system 70a, and restricts the light emitted from the light source 71. The light restriction member 73 is placed in a position that is substantially conjugated with the cornea Ec. For example, a pinhole plate or a slit plate is used as the light restriction member 73. The light restriction member 73 is used as an aperture that allows a part of the light emitted from the light source 71 to pass through and blocks the other part of the light. The light projecting optical system 70a forms a predetermined pattern light flux (for example, a spot light flux or a slit light flux) on the cornea of the eye E.

The light receiving optical system 70b includes the photodetector 77, and receives reflected light of the illuminating light from the anterior surface or posterior surface of the cornea of the eye E. The light receiving optical system 70b is placed substantially symmetrically about the optical axis L1 with respect to the light projecting optical system 70a. The light receiving optical system 70b includes, for example, a light receiving lens 75, a concave lens 76, and the photodetector 77, and forms the optical axis L3 as a light receiving optical axis. The light receiving optical system 70b in Fig. 3 shares a second working distance detecting optical system that detects the alignment state with the eye E in the Z direction.

The photodetector 77 includes a plurality of photoelectric conversion elements, and receives reflected light from each of the anterior surface and posterior surface of the cornea. For example, a light detection device such as a one-dimensional line sensor or two-dimensional area sensor is used for the photodetector 77. The magnifying power of the light receiving optical system 70b of the corneal thickness measuring optical system and the second working distance detecting optical system is increased to make an observation. Hence, the distance detection range of the photodetector 77 in the Z direction is smaller than the distance detection range of the position detector 60.

When the examinee's eye E (the cornea Ec) moves in the working distance direction (the Z direction), the reflected light of the light source 71 from the cornea Ec also moves over the photodetector 77. Therefore, the controller 80 obtains working distance information on the basis of an output signal from the photodetector 77 of the second working distance detecting optical system. Moreover, the controller 80 finds the corneal deformed state, or the blink of the examinee's eye E from the output signal from the photodetector 77, and controls the driving of the solenoid 203.

The light emitted from the illuminating light source 71 is condensed by the condenser lens 72 and illuminates the light restriction member 73 from the back. The light from the light source 71 is restricted by the light restriction member 73 and then causes the lens 53 to form an image (the light is condensed by the lens 53) near the cornea Ec. Near the cornea Ec, for example, a pinhole image (if a pinhole plate is used) or a slit image (if a slit plate is used) is formed. At this point in time, the light from the light source 71 forms an image in proximity to the point of intersection with the axis of sight on the cornea Ec.

When illuminating light is projected to the cornea Ec by the light projecting optical system 70a, reflected light of the illuminating light from the cornea Ec travels in a direction symmetrical about the optical axis L1 with respect to a light projecting light flux. The reflected light forms an image by the light receiving lens 75 on a light receiving surface of the photodetector 77.

The lens 53 shared between the light receiving optical systems 500b and 600b and the light projecting optical system 70a is placed in a position where the light of the light source 50 reflected from the cornea Ec is condensed in the central part of the hole of the pinhole plate 56 and the illuminating light from the light source 71 is condensed on the anterior surface and posterior surface of the cornea Ec.

The face photographing device 90 is, for example, an optical system for photographing the face including at least one of the examinee's left and right eyes. For example, the face photographing device 90 of the example mainly includes, for example, an imaging device 91 and an imaging lens 92 as illustrated in Fig. 3.

The face photographing device 90 is provided, for example, in a position where both eyes of the examinee can be photographed if the measuring unit 100 is in an initial position thereof. In the example, the initial position of the measuring unit 100 is set in a position displaced to the right relative to the optical axis L1 of the measuring unit 100 to facilitate the examination of the right eye. Therefore, the face photographing device 90 is provided in a position where both eyes of the examinee can be photographed while the measuring unit 100 is in the initial position displaced to the right. For example, the face photographing device 90 is placed in the machine center while the measuring unit 100 is in the initial position. If the initial position is set on the basis of, for example, half the pupillary distance, that is, the monocular pupillary distance, the face photographing device 90 may be placed in a position displaced to the left or right by the monocular pupillary distance relative to the machine center of the apparatus main body.

The face photographing device 90 of the example is moved by the driving device 4 together with the measuring unit 100. Naturally, the face photographing device 90, for example, may be configured in such a manner as to be fixed to the base 2 and unmovable.

The imaging lens 92 may be, for example, a wide-angle lens. The wide-angle lens is, for example, a fisheye lens or a conical lens. The inclusion of the wide-angle lens allows the face photographing device 90 to photograph the face of the examinee with a wide angle of view.

### <Control System>

As illustrated in Fig. 2, the non-contact tonometer 1 includes the controller 80. The controller 80 is responsible for various types of control over the non-contact tonometer 1. The controller 80 includes, for example, a general CPU (Central Processing Unit) 81, ROM 82, and RAM 83. For example, a non-contact tonometer control program for controlling the non-contact tonometer 1, and initial values are stored in the ROM 82. For example, the RAM 83 temporarily stores various pieces of information. The controller 80 is connected to, for example, the measuring unit 100, the face photographing device 90, the driving device 4, the display device 85, the operating unit 86, the chin rest driver 3d, and a storage (for example, non-volatile memory) 84. The storage 84 is, for example, a non-transitory storage medium that can hold storage contents even if the supply of power is interrupted. For example, a hard disk drive, or detachable USB flash memory can be used as the storage 84.

### <Control Operation>

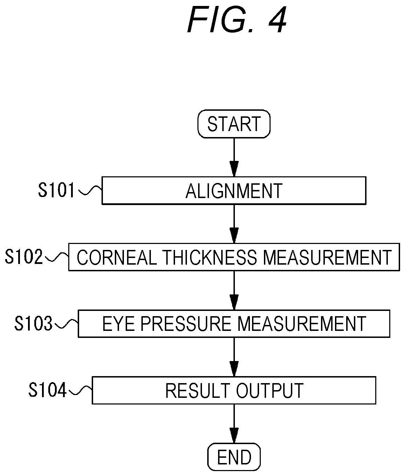

The control operation of the non-contact tonometer having the above configuration is described on the basis of Fig. 4.

### (Step S101: Alignment)

Firstly, the examiner causes the face support 3 to support the face of the examinee, and places the examinee's eye E in a predetermined position. The examiner makes alignment adjustments, operating, for example, the operating unit 86. When the alignment is completed, the examiner operates the operating unit 86 (or the controller 80 issues a measurement start signal automatically on the basis of a signal from the alignment optical system) to start a measurement.

### (Step S102: Corneal Thickness Measurement)

When the alignment is completed, the controller 80 measures the corneal thickness of the examinee's eye by use of the corneal thickness measuring optical system. The controller 80 calculates a distance between a reflected signal from the corneal anterior surface and a reflected signal from the corneal posterior surface, which have been detected by the photodetector 77 (a peak-to-peak distance).

### (Step S103: Eye Pressure Measurement)

When the measurement of the corneal thickness is completed, the controller 80 measures eye pressure. For example, when the controller 80 drives the solenoid 203 and moves the piston 202, the air in the cylinder 201 is compressed, and the compressed air is blown from the nozzle 206 to the cornea Ec. The cornea Ec deforms gradually by the blowing of the compressed air and, when the cornea Ec reaches a flat (applanated) state, the maximum amount of light enters the photosensor 57. The controller 80 obtains an eye pressure value on the basis of an output signal from the pressure sensor 212 and an output signal from the photosensor 57. The measurement result is then displayed on the display device 85. When a predetermined measurement end condition is satisfied, the eye pressure measurement of the examinee's eye is construed as complete.

### (Step S104: Result Output)

When the measurement is completed, the controller 80 outputs data on the measurement result. For example, the controller 80 causes the display device 85 to display the measurement result, prints out the measurement result, or outputs the measurement result to the outside of the apparatus in a wireless or wired manner. When the data output is completed, the controller 80 ends the process.

### <Control Operation of Fluid Ejection Device)

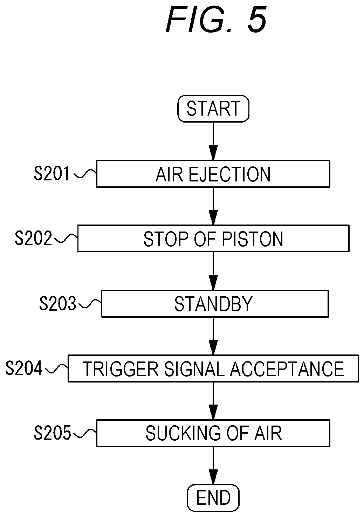

Next, the control operation of the fluid ejection device 200 during the eye pressure measurement is described on the basis of Fig. 5.

### (Step S201: Air Ejection)

Firstly, the controller 80 ejects air to the cornea of the examinee's eye. For example, the controller 80 adds an electric current as driving energy to the solenoid 203. Consequently, the solenoid 203 operates, and a driving force thereof is transmitted to the piston 202. The piston 202 moves forward in the compression direction (the direction A), and the air compressed in the cylinder 201 compresses air in the airtight chamber 221 through the tube 220. The compressed air is blown to the cornea of the examinee's eye through the nozzle 206, thereby deforming the cornea of the examinee's eye gradually.

### (Step S202: Stop of Piston)

When the controller 80 detects that the cornea has reached the applanated state on the basis of a light receiving signal of the photosensor 57, a reverse current is added to the coil 205, and a driving force (return force) in the direction B is added to the piston 202. The piston 202, which is moving in the direction A, slows down gradually due to the return force, and then stops.

### (Step S203: Standby)

The controller 80 waits with the piston 202 in the stopped state until a trigger signal for starting the sucking of air is emitted. For example, the controller 80 stops the supply of the electric current to the solenoid 203 to maintain the stopped state of the piston.

### (Step S204: Trigger Signal Acceptance)

The controller 80 accepts the trigger signal. For example, the examiner operates the operating unit 86 to output the trigger signal. For example, air is ejected to the examinee's eye, and the examiner operates the operating unit 86 to output the trigger signal for sucking air into the cylinder 201 after the surroundings of the nozzle 206 have been ventilated over a certain period of time. When having received the trigger signal outputted from the operating unit 86, the controller 80 moves on to step S205.

### (Step S205: Sucking of Air)

The controller 80 adds the return force again to the piston 202. The piston 202 is moved in the direction B by the return force, and returns to the initial position. Consequently, air is sucked into the cylinder 201. The controller 80 repeats the process from steps S201 to S205 until the eye pressure value can be measured.

As described above, in the example, the timing of sucking in the fluid can be changed on the basis of the examiner's operation. Consequently, it is possible to suck in the fluid with a timing appropriate to each measurement and to restrain lacrimal fluid, dust, or bacteria in the air from being sucked into the cylinder. For example, even if an aerosol or the like stays in the air for a certain period of time, it is possible to suck in the fluid at an appropriate timing according to, for example, the ventilation state of a measurement place.

### <Modifications>

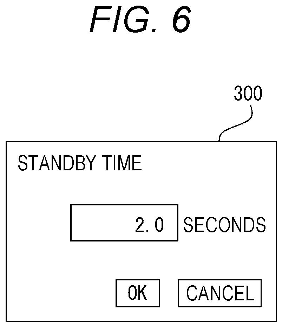

The controller 80 may start sucking in the fluid on the basis of a preset standby time. For example, the controller 80 may accept, as a trigger signal, a fact that the preset standby time has elapsed since the ejection of the fluid, and start sucking in the fluid. Consequently, the examiner does not need to operate, for example, the operating unit 86 for each ejection and output the trigger signal. Moreover, the controller 80 may change the standby time. For example, the controller 80 may change the standby time on the basis of an operation signal outputted by the examiner's operation from the operating unit 86. For example, the controller 80 displays a standby time setting screen 300 on the display device 85 as illustrated in Fig. 6. In the case of Fig. 6, the current standby time is being displayed on the setting screen 300. For example, the examiner selects the number of seconds of the current standby time, inputs a new numerical value, and presses an OK button. In this manner, the examiner can change the standby time, and set a new standby time.

The controller 80 may be configured in such a manner as to be capable of switching a plurality of operation modes having different timing change conditions. For example, the controller 80 may switch between a manual change mode (a first mode) in which the timing of sucking is changed manually and a time setting mode (a second mode) in which sucking is started at a timing when a preset standby time has elapsed. As described in the above example, the manual change mode is a mode in which for each ejection, the examiner operates, for example, the operating unit 86 to generate the trigger signal, thereby starting the sucking operation. In the manual change mode, the sucking operation can be started according to each measurement situation. The time setting mode is, for example, a mode in which a period of time from after ejection to the start of the sucking operation is preset, and the sucking operation is started automatically after the preset period of time has elapsed since the ejection. In the time setting mode, there is no need to operate the operating unit 86 for each ejection as long as the period of time is preset.

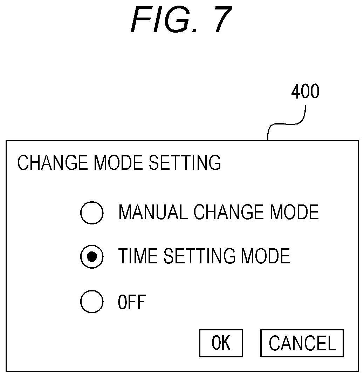

For example, the display device 85 may be caused to display a mode setting screen 400, and the examiner may be caused to select the operation mode, as illustrated in Fig. 7. In this case, the controller 80 switches the operation mode to an operation mode selected by the examiner. In this manner, a plurality of operation modes having different timing change conditions is made switchable; therefore, a measurement can be made in an operation mode that is easy for the examiner to use.

The controller 80 may be configured in such a manner as to be capable of turning OFF the change function. In this case, the controller 80 waits for a predetermined period of time after stopping the piston 202, and then starts sucking in the fluid. In this manner, the controller 80 may switch between the mode of changing the timing of sucking of the fluid and the mode of sucking in at a predetermined timing without changing the timing of sucking in the fluid. A change in the timing of sucking brings about the possibility of increasing an examination time; therefore, a setting appropriate for each facility can be made by switching between the enabling and disabling of the change function.

The controller 80, for example, may detect the presence or absence of the examinee by use of a detector such as the chin rest sensor 3c or the face photographing device 90, and change the timing of sucking in the fluid according to the result of the detection. For example, the controller 80 may be configured in such a manner as to suck in the fluid when it has been determined that the face of the examinee has moved away from the face support 3 on the basis of the detection result of, for example, the chin rest sensor 3c or the face photographing device 90. The face photographing device 90 detects the presence or absence of the examinee and therefore can indirectly detect the possibility of sucking a foreign substance into the cylinder 201.

Moreover, the controller 80 may change the timing of sucking in the fluid on the basis of the position of the measuring unit 100. For example, the non-contact tonometer 1 may include a position sensor that detects the position of the measuring unit 100. For example, the position sensor may detect that the measuring unit 100 is in an origin position (initial position). The controller 80 may be configured in such a manner as to start sucking in the fluid when the position sensor has detected that the measuring unit 100 is in the origin position. For example, the controller 80 may cause the position sensor to detect that the measuring unit 100 has been moved back to the origin position by the examiner after the ejection of the fluid, and then suck in the fluid. The position sensor detects the position of the measuring unit 100 and therefore the possibility of sucking a foreign substance into the cylinder 201 can be indirectly detected.

Instead of the position sensor, a range sensor may be provided. For example, the measuring unit 100 may be provided on an examinee side thereof with the range sensor, and change the timing of sucking in the fluid on the basis of a distance to the examinee (or the face support 3) measured by the range sensor. The range sensor detects the distance to the examinee; therefore, the possibility of sucking a foreign substance into the cylinder 201 can be indirectly detected.

The non-contact tonometer 1 may include a nozzle retraction mechanism. The nozzle retraction mechanism is for moving the tip of the nozzle away from the examinee's eye. The nozzle retraction mechanism, for example, may retract (store) the nozzle in the measuring unit 100. In this case, for example, the controller 80 may be configured in such a manner as to start sucking in the fluid after the nozzle 206 is retracted. The nozzle retraction mechanism may be for retracting the nozzle 206 by moving the nozzle 206 in the Z direction, the X direction, or the Y direction, or may retract the nozzle 206 by rotating the nozzle 206 around a horizontal axis or a vertical axis. Moreover, the non-contact tonometer 1 may include a nozzle position sensor that detects the position of the nozzle 206. In this case, the controller 80 may be configured in such a manner as to start sucking in the fluid after the nozzle position sensor detects that the nozzle 206 has been retracted.

The controller 80 may be configured in such a manner as to detect the blink of the examinee on the basis of the pupil, or a raster, appearing in an anterior segment image that is captured by the observing optical system (the CCD camera 35) and then start sucking in the fluid while the blinks of the examinee subside. The controller 80, for example, may suck in the fluid after several seconds elapse since the detection of the final blink. Consequently, it is possible to restrain the lacrimal fluid flown by the blink from being sucked in. The observing optical system detects the blink of the examinee; therefore, the possibility of sucking a foreign substance into the cylinder 201 can be indirectly detected.

The controller 80 may control the fluid ejection device 200 when the examinee is changed, and eject the fluid several times when the examinee is absent. Consequently, even if, for example, lacrimal fluid, dust, or a bacterium is trapped in the cylinder, the air in the cylinder can be ventilated.

### <Second Example>

A second example is described below. A non-contact tonometer of the second example is different in the control operation of the fluid ejection device from the first example. The apparatus configuration is similar to the first example, and therefore a description thereof is omitted.

### <Control Operation of Fluid Ejection Device>

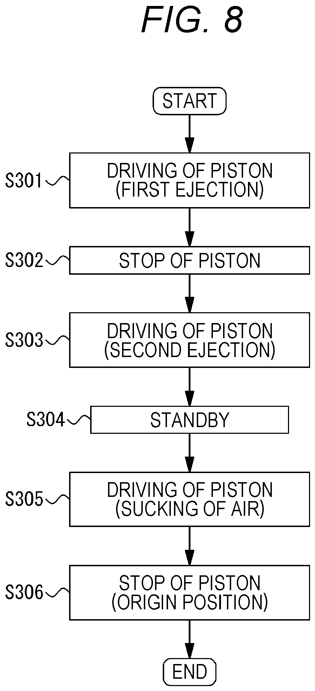

The control operation of the fluid ejection device 200 during eye pressure measurement of the second example is described on the basis of Fig. 8. The non-contact tonometer 1, for example, ejects air (performs a second ejection) to blow off and remove a foreign substance such as an aerosol floating near the nozzle 206, separately from an ejection (a first ejection) for deforming the cornea. Examples of the aerosol include lacrimal fluid on the cornea of an examinee's eye, attached substances of surrounding tissue, viruses and bacteria, which are flown by the first ejection.

### {Step S301: Driving of Piston (First Ejection)}

Firstly, the controller 80 ejects air to the cornea of the examinee's eye. For example, the controller 80 supplies an electric current as driving energy to the solenoid 203. Consequently, the solenoid 203 operates, and a driving force thereof is transmitted to the piston 202. The piston 202 is moved forward in the direction A. Air compressed in the cylinder 201 compresses air in the airtight chamber 221 through the tube 220. The compressed air is then blown to the cornea of the examinee's eye through the nozzle 206 to deform the cornea of the examinee's eye.

### {Step S302: Stop of Piston}

When having detected that the cornea has reached the applanated state on the basis of a light receiving signal of the photosensor 57, the controller 80 supplies a reverse current to the coil 205, and adds a driving force (return force) in the direction B to the piston 202. The piston 202, which is moving in the direction A, slows down gradually due to the return force, and stops.

### {Step S303: Driving of Piston (Second Ejection)}

After stopping the piston 202, the controller 80 supplies an electric current again to the coil 205, and moves the piston 202 in the direction A while a force is being added to the piston 202 (the piston 202 is being biased). Consequently, air is ejected again to the examinee's eye. The controller 80 may move (bias) the piston 202 in the direction A up to a movement limit position (a position where the front surface of the piston 202 comes into contact with the cylinder).

### (Step S304: Standby)

The controller 80 waits with the piston 202 in a stopped state until receiving a trigger signal for starting sucking in air. For example, the controller 80 stops the supply of the electric current to the solenoid 203 to maintain the stopped state of the piston 202.

### {Step S305: Driving of Piston (Sucking of Air)}

The controller 80 adds the return force again to the piston 202. The return force moves the piston 202 in the direction B. Consequently, air is sucked into the cylinder 201.

### {Step S306: Stop of Piston (Origin Position)}

The controller 80 stops the supply of the electric current when the piston 202 stops in the origin position. The controller 80 repeats the process from steps S301 to S306 until an eye pressure value can be measured.

The above non-contact tonometer 1 needs to return the piston 202 to the origin position and suck air into the cylinder for the next measurement. However, a foreign substance such as an aerosol may float near the nozzle immediately after the measurement. Hence, the non-contact tonometer 1 of the example ejects air again after ejecting air for measurement. In this manner, it is possible to restrain a foreign substance such as an aerosol from being sucked in from the nozzle 206 by the sucking operation due to the performance of the second ejection after the first ejection.

As in the example, the second ejection is performed at a timing before the air sucking operation is completed; therefore, it is possible to blow and remove a foreign substance such as an aerosol before the sucking of air is completed. As a result, it is possible to reduce the possibility of sucking the foreign substance into the cylinder 201.

It is not necessary to execute the first ejection and the second ejection in a series of sequence. The first ejection and the second ejection may be executed independently of each other at any timings. For example, the controller 80 may end the measurement operation after performing the first ejection, and then perform the second ejection at any timing regardless of the first ejection.

It is preferable that the pressure of the fluid in the second ejection be lower than the pressure of the fluid in the first ejection. For example, the first ejection is performed at a high pressure so that the cornea can be deformed. However, the second ejection is simply required to be able to blow off, for example, an aerosol near the nozzle 206, and is performed at a low pressure to prevent, for example, a new aerosol from being generated.

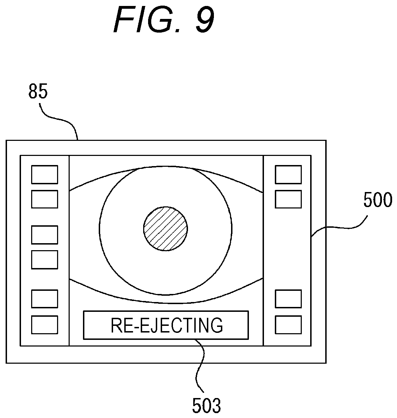

The controller 80 may cause the display device 85 to display the performance of the second ejection. For example, the controller 80 may display a mark 503 of the second ejection (re-ejection) on a measurement screen 500 displayed on the display device 85 as illustrated in Fig. 9. Consequently, the examiner can confirm that the second ejection is being performed. Moreover, the examiner can explain to, for example, the examinee that the second ejection is being performed.

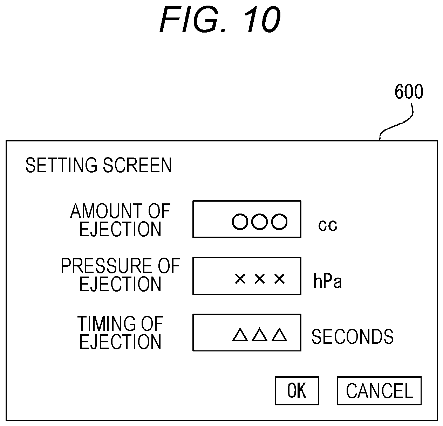

The controller 80 may change, for example, the amount, pressure, or timing of ejection in the second ejection. For example, it may be configured in such a manner that the examiner can set the amount, pressure, or timing of ejection freely by use of a setting screen 600 displayed on the display device 85, as illustrated in Fig. 10. The controller 80, for example, may change the magnitude of an electric current that is supplied to the solenoid, a supply time of the electric current, or the like on the basis of the set amount, pressure, or timing of ejection.

In the above examples, the controller 80 temporarily stops the piston 202 after performing the first ejection. However, the present disclosure is not limited to this. For example, the controller 80 may continue performing the second ejection gently in such a manner that the ejection is carried over to the first ejection. For example, the controller 80 may perform the second ejection without stopping the piston 202. In this case, for example, the controller 80 may add the return force in the direction B to the piston 202, which is moving in the direction A in the first ejection, to slow down the piston 202, cancel the return force before the piston 202 stops, add the force in the direction A again to bias the piston 202, and perform a re-ejection without stopping the piston 202.

In the above examples, the controller 80 performs the second ejection by driving the solenoid 203. However, the present disclosure is not limited to this. For example, a point that a metal plate 210 (refer to Fig. 2) that supports the cylinder 201, and the movable body 204 are attracted to each other by a magnetic force may be used to bias the piston 202. For example, after the first ejection, the controller 80 removes the return force caused by the solenoid 203 before completely stopping the piston 202 with the return force caused by the solenoid 203. The piston 202 continues moving under its own inertia. When the movable body 204 is moved to a position where the movable body 204 and the metal plate 210 are attracted to each other by the magnetic force, the piston 202 is biased by the magnetic force in the direction A, and moved to perform the second ejection. In this manner, not only the driving of the solenoid 203 but also another biasing means may bias the piston 202 to perform the second ejection.

The controller 80 may be configured in such a manner as to switch between the performance and non-performance of the second ejection. If the function of the second ejection is turned OFF, for example, the controller 80 moves on to the standby operation in step S304 without performing the second ejection operation in step S303. In this manner, the performance and non-performance of the second ejection is made switchable; therefore, a measurement can be made with an appropriate setting according to each examiner or each facility.

In the above examples, the case where a direct acting solenoid is used as the solenoid 203 has been described. However, a rotary solenoid, or another driving source, may be used as the solenoid 203.

### LIST OF REFERENCE SIGNS

- 1: Non-contact tonometer
- 80: Controller
- 201: Cylinder
- 202: Piston
- 203: Solenoid

## Claims

1. A non-contact tonometer that measures eye pressure in an examinee's eye in a non-contact manner, the non-contact tonometer comprising:
a fluid ejection means for ejecting fluid to a cornea of the examinee's eye;
a driving means for driving the fluid ejection means; and
a control means for controlling the driving means,
wherein the control means changes a timing of starting a fluid sucking operation after ejection of the fluid.

2. The non-contact tonometer according to claim 1, wherein the control means changes a setting of a standby time after the ejection of the fluid to change the timing.

3. The non-contact tonometer according to claim 1 or 2, further comprising an operation accepting means for accepting an operation of an examiner,
wherein the control means starts the fluid sucking operation based on an operation of the examiner accepted by the operation accepting means during standby after the ejection of the fluid.

4. The non-contact tonometer according to any one of claims 1 to 3, further comprising a detection means,
wherein the control means changes the timing based on a detection result of the detection means.

5. The non-contact tonometer according to any one of claims 1 to 4, wherein the control means switches between a first mode of changing the timing and a second mode of changing the timing based on a condition different from the first mode.

6. The non-contact tonometer according to any one of claims 1 to 5, wherein the control means switches whether or not to change the timing.

7. A non-contact tonometer that measures eye pressure in an examinee's eye in a non-contact manner, the non-contact tonometer comprising:
a fluid ejection means for ejecting fluid to a cornea of the examinee's eye;
a driving means for driving the fluid ejection means; and
a control means for controlling the driving means,
wherein the control means waits until a possibility of sucking in a foreign substance after ejection of the fluid is reduced, and then starts a fluid sucking operation.

8. A non-contact tonometer that measures eye pressure in an examinee's eye in a non-contact manner, the non-contact tonometer comprising:
a fluid ejection means for ejecting fluid to a cornea of the examinee's eye;
a driving means for driving the fluid ejection means; and
a control means for controlling the driving means,
wherein the control means performs a first ejection for deforming the cornea during eye pressure measurement and, after the first ejection, performs a second ejection for removing a foreign substance near an outlet of the fluid ejection means before a next measurement.

9. The non-contact tonometer according to claim 8, wherein the control means performs the second ejection before a fluid sucking operation is completed.

10. The non-contact tonometer according to claim 8 or 9, wherein
the fluid ejection means includes a cylinder, and a piston that moves in the cylinder, and
after biasing the piston, which is moving in a forward direction of movement in the first ejection, in a backward direction of movement to slow down or stop the piston, the control means biases the piston again in the forward direction of movement to perform the second ejection.

11. The non-contact tonometer according to any one of claims 8 to 10, wherein pressure of the fluid ejected in the second ejection is lower than pressure of the fluid ejected in the first ejection.

12. The non-contact tonometer according to any one of claims 8 to 11, wherein the control means displays, on a display means, information that the second ejection is being performed.

13. The non-contact tonometer according to any one of claims 8 to 12, wherein the control means changes a timing of starting the fluid sucking operation after ejection of the fluid.

14. A tonometer control program to be executed in a non-contact tonometer that measures eye pressure in an examinee's eye in a non-contact manner, the tonometer control program being executed by a control means of the non-contact tonometer, thereby causing the non-contact tonometer to execute:
a driving step of driving a fluid ejection means for ejecting fluid to a cornea of the examinee's eye; and
a changing step of changing a timing of starting a fluid sucking operation after ejection of the fluid.

15. A tonometer control program to be executed in a non-contact tonometer that measures eye pressure in an examinee's eye in a non-contact manner, the tonometer control program being executed by a control means of the non-contact tonometer, thereby causing the non-contact tonometer to execute:
a driving step of driving a fluid ejection means for ejecting fluid to a cornea of the examinee's eye;
a first ejection step of performing a first ejection for deforming the cornea during eye pressure measurement; and
a second ejection step of, after the first ejection, performing a second ejection for removing a foreign substance near an outlet of the fluid ejection means before a next measurement.
